# EUROPEAN PATENT SPECIFICATION

(11) **EP 3 518 911 B1**
(45) Date of publication and mention of the grant of the patent: **25.08.2021**
(21) Application number: 17857371.3
(22) Date of filing: 27.09.2017
(51) Int. Cl.: A61K 31/137, A61K 31/337, A61K 31/506, A61K 31/519, A61K 45/06, A61P 35/00, A61P 35/04, C07C 217/84

(54) **RAD1901 FOR USE IN TREATING OVARIAN CANCER**
RAD1901 ZUR VERWENDUNG BEI DER BEHANDLUNG VON OVARIALKARZINOM
RAD1901 POUR UTILISATION DANS LE TRAITEMENT DU CANCER DE L'OVAIRE

(30) Priority: 27.09.2016 US 201662400495 P
(43) Date of publication of application: 07.08.2019
(73) Proprietor: Radius Pharmaceuticals, Inc., Boston, MA 02210 (US)
(72) Inventor: HATTERSLEY, Gary, Stow MA 01775 (US)
(74) Representative: Potter Clarkson
(86) International application number: PCT/US2017/053834
(87) International publication number: WO 2018/064231

(56) References cited:
- WO-A1-2016/176664
- WO-A1-2016/176665
- WO-A1-2016/176666
- US-A1- 2007 213 543
- US-A1- 2011 009 387
- US-A1- 2015 231 134
- US-A1- 2015 274 640
- Anonymous: "Radius (RDUS) Announced Today That It Has Acquired The License To Develop And Market RAD1901 In Japan", MPM - Globe Newswire, 10 March 2015 (2015-03-10), pages 1-5, XP055683544, Retrieved from the Internet: URL:https://www.mpmcapital.com/press/radiu s-rdus-announced-today-acquired-license-de velop-market-rad1901-japan/ [retrieved on 2020-04-07]

## Description

### PRIORITY CLAIM

This application claims the benefit of U.S. Provisional Application No. 62/400,495, filed September 27, 2016.

### BACKGROUND

Ovarian tumors are known to often express estrogen receptor (ER) including ERα. ERs can be activated by estrogen and translocate into the nucleus to bind to DNA, thereby regulating the activity of various genes. See, e.g., Marino et al., "Estrogen Signaling Multiple Pathways to Impact Gene Transcription," Curr. Genomics 7(8): 497-508 (2006); and Heldring et al., "Estrogen Receptors: How Do They Signal and What Are Their Targets," Physiol. Rev. 87(3): 905-931 (2007). Ovarian cancers may be of different types including epithelial, stromal and sarcomas. Epithelial ovarian cancers are typically divided into serous, endometrioid, mucinous, clear cell and undifferentiated. Ovarian cancers may also be primary peritoneal, stromal and sarcomas. Regardless of origin or type, ovarian cancer is a serious and often fatal affliction with a great recognized need for new and effective treatment modalities. US-A-2015/0274640 discloses a selective estrogen receptor modulator, preferably RAD1901, for use in the treatment of cancer that is resistant to an estrogen receptor modulator.

### BRIEF SUMMARY OF THE INVENTION

The invention relates to a compound for use in inhibiting tumor growth or producing tumor regression in a subject having ovarian cancer or tumor selected from the group consisting of epithelial ovarian cancer, stromal ovarian cancer and ovarian sarcoma cancer, wherein said compound is RAD1901 or a salt or solvate thereof.

In an aspect of the invention, said use comprises administering to the subject a therapeutically effective amount of RAD1901 of RAD1901 and a therapeutically effective amount of one or more second therapeutic agents.

In certain embodiments of the invention, the ovarian cancer and/or tumor treated expresses ERα.

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

### BRIEF DESCRIPTION OF DRAWINGS AND TABLES

**Figure 1****:** Tumor growth inhibition and regression effects of RAD1901 (p.o. daily at 30 mg/kg or 60 mg/kg) to PDX models (mice with established ovarian patient derived xenograft) tumors).
**Figure 2****:** Individual tumor growth inhibition and regression effect at day 32 in PDX models treated with RAD1901 (p.o., daily at 30 mg/kg or 60 mg/kg).

### DETAILED DESCRIPTION OF THE INVENTION

Given the generally poor long term prognoses for ovarian cancer there is an urgent need for new and particularly efficacious agents that target heretofore unapproved pathways for treatment. There are currently no officially indicated antiestrogens or selective estrogen receptor degraders (SERDs) FDA approved for the treatment of ovarian cancer making the need for a relatively non-toxic, well tolerated drug effective through the estrogen receptor a particularly exciting and important breakthrough. RAD1901 was found to inhibit tumor growth and/or drive tumor regression in breast cancer xenograft models, regardless of ESR1 mutation status and prior endocrine therapy. In the examples provided herein, RAD1901 was shown to be effective to inhibit tumor growth and unexpectedly drive tumor regression in certain PDX models (mice bearing a patient derived ovarian cancer xenograft) at a daily oral dosage of 30 mg/kg or 60 mg/kg.

As used herein, RAD1901 has the following structure, 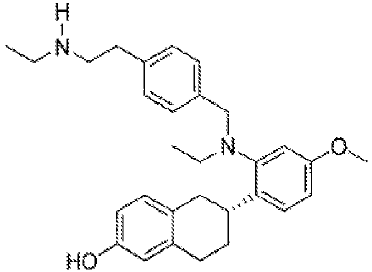 including salts or solvates (e.g. hydrates) thereof.

In certain embodiments, the invention relates to RAD1901 or a salt or solvate thereof for use in inhibiting tumor growth or producing tumor regression in a subject having ovarian cancer or tumor selected from the group consisting of epithelial ovarian cancer, stromal ovarian cancer and ovarian sarcoma cancer.

In certain embodiments, methods are provided for inhibiting growth or producing regression of ovarian cancer or tumor in a subject in need thereof by administering to the subject a therapeutically effective amount of RAD1901 as disclosed herein (e.g.. or a salt or solvate (e.g., hydrate) thereof).

In certain embodiments of the methods disclosed herein, the ovarian cancer or tumor is ERa positive.

In certain embodiments of the methods disclosed herein, the salt of RAD1901 is RAD1901 dihydrochloride having the following structure:

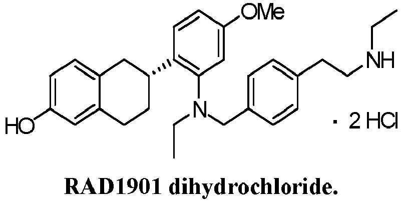

"Treating" of an ovarian cancer or tumor as used herein refers to achievement of one or more therapeutic benchmarks, including, without limitation, slowing or halting of tumor growth, driving tumor regression, reduction or cessation of one or more symptoms, reducing chances of having ovarian cancer or tumor, reducing the possibility or prolonging the time for a re-occurrence of ovarian cancer or tumor, etc.

"Inhibiting growth" of an ovarian cancer or tumor as used herein refers to slowing the rate of tumor growth, or halting tumor growth entirely.

"Tumor regression" or "regression" of an ovarian cancer or tumor as used herein refers to reducing the maximum size of the ovarian cancer or tumor.

In certain embodiments, administration of RAD1901 as disclosed herein (e.g.. or a salt or solvate (e.g., hydrate) thereof) to a subject having an ovarian cancer or tumor may result in a decrease in the ovarian cancer or tumor size versus baseline (i.e., size prior to initiation of treatment), or even eradication or partial eradication of the ovarian cancer or tumor. Accordingly, certain embodiments of the methods disclosed herein result in reducing the ovarian cancer burden or tumor size of one or more tumors versus pretreatment baseline.

"Tumor" as used herein is malignant tumor, and is used interchangeably with "cancer."

Tumor growth inhibition or regression may be localized to a single tumor or to a set of tumors within a specific tissue or organ, or may be systemic (i.e., affecting tumors in all tissues or organs).

As RAD1901 is known to preferentially bind ERa versus estrogen receptor beta (ERβ), unless specified otherwise, estrogen receptor, estrogen receptor alpha, ER-α, ERα, ER, wild-type ER-α, and ESR1 are used interchangeably herein. "Estrogen receptor alpha" or "ERα" is encoded by the gene *ESR1.* An ovarian cancer or tumor that is "positive for estrogen receptor alpha," "ERα-positive," "ER+," or "ERα+" as used herein refers to an ovarian cancer or tumor in which one or more cells express at least one isoform of ERa. In certain embodiments, these cells overexpress ERα.

In certain embodiments of the methods disclosed herein, the ovarian cancer or tumor is resistant to a drug selected from the group consisting of anti-estrogens (e.g., tamoxifen or fulvestrant), aromatase inhibitors (e.g., aromasin), and combinations thereof.

In general, the methods disclosed herein are applicable to the treatment of an ovarian-originating tumor whether that tumor is located in the ovary or outside of the ovary (including elsewhere in the body) or both. For example, in some embodiments the primary ovarian cancer or tumor of the subject has already been surgically removed (e.g., by tumor excision or organ excision) and the subject is treated for 1) remaining metastatic ovarian cancer or tumor outside of one or more of her ovaries; and/or 2) reducing the possibility or prolonging the time for a re-occurrence. In some embodiments, the subject treated still has the primary ovarian cancer or tumor in one or more ovaries when the therapy is initiated.

In certain embodiments of the methods disclosed herein, RAD1901 is administered prophylactically to women deemed to be at high risk for ovarian cancer or tumor through one or more markers, characteristics, history or features that risk is ascertained. In such instances, the subject is medically determined to be at an elevated risk for ovarian cancer or tumor and RAD1901 is given to reduce the chances of the woman getting ovarian cancer or tumor.

In certain embodiments of the methods disclosed herein, the ovarian cancer or tumor being targeted is an epithelial ovarian cancer or tumor. In certain embodiments of the methods disclosed herein, the ovarian cancer or tumor is an epithelial ovarian cancer or tumor with a high grade serous, low grade serous, serous, endometrioid, mucinous, clear cell, primary peritoneal, brenner tumours, borderline tumours, or of undifferentiated type. In some embodiments the ovarian cancer to be treated is a stromal cancer or tumor. In some embodiments the ovarian cancer to be treated is an ovarian sarcoma.

In certain embodiments of the methods provided herein, the methods further comprise a step of determining whether a patient has an ovarian cancer or tumor expressing ERa prior to administering RAD1901 or a salt or solvate thereof, for example the bis-hydrochloride salt (2HCls per molecule of RAD1901). In certain embodiments the tumor to be treated expresses ER in >90%, >80%, >70%, >60%, >50%, >40%, >30%, >20%, >10%, >5%, or >1% of the cells in the ovarian cancer or tumor.

A therapeutically effective amount of RAD1901 for use in the methods disclosed herein is an amount that, when administered over a particular time interval, results in achievement of one or more therapeutic benchmarks (e.g., slowing or halting of tumor growth, driving tumor regression, cessation or reduction of one or more symptoms, reducing chances of having ovarian cancer or tumor, reducing the possibility or prolonging the time for a re-occurrence of ovarian cancer or tumor, etc.).

Examples of therapeutically effective amounts of RAD1901 for use in the methods disclosed herein include, without limitation, about 150 mg to about 2,000 mg, about 200 mg, about 400 mg, about 500 mg, or about 1,500 mg. More particularly the RAD1901 may be administered in a daily dosage amount of about 100 mg to about 1,000 mg, about 200 mg, about 400 mg, about 500 mg, about 600 mg, about 800 mg, or about 1,000 mg. In certain embodiments of the methods disclosed herein, the RAD1901 administered is a bis-hydrochloride of RAD1901 (2 HCls per molecule of RAD1901).

RAD1901 disclosed herein (and/or salts or solvates thereof) for use in the presently disclosed methods can be formulated into unit dosage forms, which are physically discrete units suitable as unitary dosage for subjects undergoing treatment. Each unit dosage contains a therapeutically effective amount of RAD1901, optionally in association with a suitable pharmaceutical carrier. The unit dosage form can be for a single daily dose or one for multiple daily doses (e.g., about 1 to 4 or more times q.d.). When multiple daily doses are used, the unit dosage form can be the same or different for each dose. In certain embodiments, RAD1901 disclosed herein may be formulated for controlled release.

RAD1901 disclosed herein (and/or salts or solvates thereof) for use in the presently disclosed methods can be formulated into a pharmaceutical composition as any the active ingredient. The pharmaceutical composition of RAD1901 may further comprises a physiologically acceptable carrier (also referred to as a pharmaceutically acceptable carrier or solution or diluent). Such pharmaceutical compositions are prepared in accordance with acceptable pharmaceutical procedures such as described in Remington's Pharmaceutical Sciences, 17th edition, ed. Alfonso R. Gennaro, Mack Publishing Company, Eaton, Pa. (1985).

The term "pharmaceutically acceptable carrier" refers to a carrier that does not cause an allergic reaction or other untoward effect in subjects to whom it is administered and are compatible with the other ingredients in the formulation. Pharmaceutically acceptable carriers include, for example, pharmaceutical diluents, excipients or carriers suitably selected with respect to the intended form of administration, and consistent with conventional pharmaceutical practices. For example, solid carriers/diluents include, but are not limited to, a gum, a starch (e.g., corn starch, pregelatinized starch), a sugar (e.g., lactose, mannitol, sucrose, dextrose), a cellulosic material (e.g., microcrystalline cellulose), an acrylate (e.g., polymethylacrylate), calcium carbonate, magnesium oxide, talc, or mixtures thereof. Pharmaceutically acceptable carriers may further comprise minor amounts of auxiliary substances such as wetting or emulsifying agents, preservatives or buffers, which enhance the shelf life or effectiveness of the therapeutic agent.

RAD1901 in a free base form can be converted into a salt by conventional methods. The term "salt" used herein is not limited as long as the salt is formed with RAD1901 in a free base and is pharmacologically acceptable; preferred examples of salts include a hydrohalide salt (for instance, hydrochloride, hydrobromide, hydroiodide and the like), an inorganic acid salt (for instance, sulfate, nitrate, perchlorate, phosphate, carbonate, bicarbonate and the like), an organic carboxylate salt (for instance, acetate salt, maleate salt, tartrate salt, fumarate salt, citrate salt and the like), an organic sulfonate salt (for instance, methanesulfonate salt, ethanesulfonate salt, benzenesulfonate salt, toluenesulfonate salt, camphorsulfonate salt and the like), an amino acid salt (for instance, aspartate salt, glutamate salt and the like), a quaternary ammonium salt, an alkaline metal salt (for instance, sodium salt, potassium salt and the like), an alkaline earth metal salt (magnesium salt, calcium salt and the like) and the like. In addition, hydrochloride salt, sulfate salt, methanesulfonate salt, acetate salt and the like are preferred "pharmacologically acceptable salt" of RAD1901 for the methods disclosed herein.

Isomers of RAD1901 disclosed herein (e.g., geometric isomers, optical isomers, rotamers, tautomers) can be purified using general separation means, including for example recrystallization, optical resolution such as diastereomeric salt method, enzyme fractionation method, various chromatographies (for instance, thin layer chromatography, column chromatography, glass chromatography and the like) into a single isomer. The term "a single isomer" herein includes not only an isomer having a purity of 100%, but also an isomer containing an isomer other than the target, which exists even through the conventional purification operation. A crystal polymorph sometimes exists for RAD1901 or a salt thereof, and all crystal polymorphs thereof are included in the present invention. The crystal polymorph of RAD1901 may be a single type of crystal polymorph or a mixture of multiple crystal polymorphs of RAD1901.

In certain aspects of the disclosure, RAD1901 is in a prodrug form which will be converted (e.g., via oxidation or hydrolysis) to an active form of RAD1901.

In certain embodiments, the methods provided herein further comprise gene profiling the subject, wherein the gene to be profiled is one or more genes selected from the group consisting of ABL1, AKT1, AKT2, ALK, APC, AR, ARID1A, ASXL1, ATM, AURKA, BAP, BAP1, BCL2L11, BCR, BRAF, BRCA1, BRCA2, CCND1, CCND2, CCND3, CCNE1, CDH1, CDK4, CDK6, CDK8, CDKN1A, CDKN1B, CDKN2A, CDKN2B, CEBPA, CTNNB1, DDR2, DNMT3A, E2F3, EGFR, EML4, EPHB2, ERBB2, ERBB3, ESR1, EWSR1, FBXW7, FGF4, FGFR1, FGFR2, FGFR3, FLT3, FRS2, HIF1A, HRAS, IDH1, IDH2, IGF1R, JAK2, KDM6A, KDR, KIF5B, KIT, KRAS, LRP1B, MAP2K1, MAP2K4, MCL1, MDM2, MDM4, MET, MGMT, MLL, MPL, MSH6, MTOR, MYC, NF1, NF2, NKX2-1, NOTCH1, NPM, NRAS, PDGFRA, PIK3CA, PIK3R1, PML, PTEN, PTPRD, RARA, RBI, RET, RICTOR, ROS1, RPTOR, RUNX1, SMAD4, SMARCA4, SOX2, STK11, TET2, TP53, TSC1, TSC2, and VHL.

In certain embodiments, the methods provided herein further comprise adjusting the dosage of RAD1901 disclosed herein (and/or salt (e.g., HCl salt) or solvate (e.g., hydrate) thereof) comprising:
(1) administering a first dosage of RAD1901 disclosed herein (and/or salt (e.g., HCl salt) or solvate (e.g., hydrate) thereof) (e.g., about 350 to about 500 mg/day) for 3, 4, 5, 6, or 7 days;
(2) detecting estradiol-ER binding activity, for example using FES-PET imaging as disclosed herein; wherein:
   (i) if the ER binding activity is not detectable or is below a predetermined threshold level, continuing to administer the first dosage (i.e., maintain the dosage level); or
   (ii) if the ER binding activity is detectable or is above a predetermined threshold level, administering a second dosage that is greater than the first dosage (e.g., the first dosage plus about 50 to about 200 mg) for 3, 4, 5, 6, or 7 days, then proceeding to step (3);
(3) detecting estradiol-ER binding activity, for example using FES-PET imaging as disclosed herein; wherein
   (i) if the ER binding activity is not detectable or is below a predetermined threshold level, continuing to administer the second dosage (i.e., maintain the dosage level); or
   (ii) if the ER binding activity is detectable or is above a predetermined threshold level, administering a third dosage that is greater than the second dosage (e.g., the second dosage plus about 50 to about 200 mg) for 3, 4, 5, 6, or 7 days, then proceeding to step (4);
(4) repeating the steps above through a fourth dosage, fifth dosage, etc., until no ER binding activity is detected.

Certain embodiments of the methods disclosed herein further include the use of PET imaging to detect and/or dose ER sensitive or ER resistant cancers.

The term "and/or" as used herein includes both the "and" case and the "or" case.

Certain embodiments of the methods disclosed herein further comprises administering to the subject a therapeutically effective amount of one or more second therapeutic agents selected from the group consisting of CDK4 and/or CDK6 inhibitor(s) (e.g., ribociclib, abemaciclib and palbociclib), taxane (.g., paclitaxel, albumin bound paclitaxel (e.g., abraxane), docetaxel, cabazitaxel), altretamine, capecitabine, etoposide, gemcitabine, ifosfamide, irinotecan, doxorubicin, liposomal doxorubicin, mephalen, pemetrexed, topotecan, vinorelbine, and PI3K inhibitors (e.g., idelalisib, perifosine, buparlisib, duvelisib, alpelisib, TGR1202, copanlisib, px-866, dactolisib, RP6530, SF1126, INK1117, pictilisib, XL147, XL765, palomid 529, ZSTK474, PWT33597, CUDC 907, ME401, IPI549, IC87114, TG100-115, CAL263, RP6503, PI103, GNE477, AEZS136). When more than one therapeutic agents are administered, the multiple therapeutic agents may be administered at the same time or substantially at the same time as one or more combined or separate pharmaceutical composition. Alternatively, the more than one therapeutic agents may be administered at different time by one controlled release pharmaceutical composition or by separate pharmaceutical composition.

The following examples are provided to better illustrate the claimed invention and are not to be interpreted as limiting the scope of the invention. To the extent that specific materials are mentioned, it is merely for purposes of illustration and is not intended to limit the invention.

### Examples

### Materials and methods

RAD1901 used in the examples below was (6R)-6-(2-(N-(4-(2-(ethylamino)ethyl)benzyl)-N-ethylamino)-4-methoxyphenyl)-5,6,7,8-tetrahydronaphthalen-2-ol dihydrochloride, manufactured by IRIX Pharmaceuticals, Inc. (Florence, SC). RAD1901 was stored as a dry powder, formulated for use as a homogenous suspension in 0.5% (w/v) methylcellulose in deionized water, and for animal models was administered p.o.

### In vivo Xenograft PDX Models

All mice were housed in pathogen-free housing in individually ventilated cages with sterilized and dust-free bedding cobs, access to sterilized food and water ad libitum, under a light dark cycle (12-14 hour circadian cycle of artificial light) and controlled room temperature and humidity.

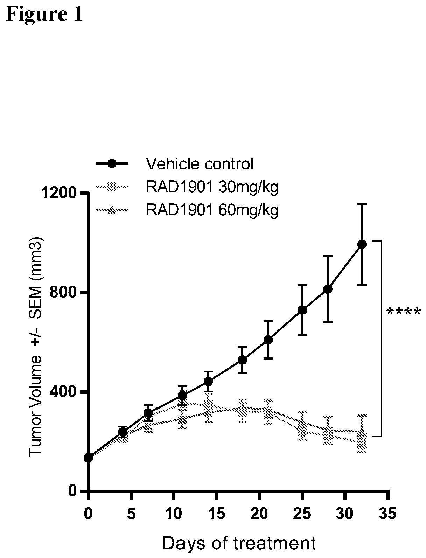

The PDX shown in Figure 1 was the OV-10-0050 ovarian cancer tumor, derived from an ovarian adenocarcinoma from a 48 year woman. The tumor was grade 3 and positive for cytokeratin indicating an ovarian epithelial type. The tumor also stained positive for ER and an ER target gene (progesterone receptor). The OV-10-0050 PDX model was established from viable human tumor tissue that had been serially passaged in animals (female Balb/c nude mice) a limited number of times to maintain tumor heterogeneity. When tumors reached the appropriate Tumor Volume Initiation (TVI) range (150-250 mm³), animals were randomized into treatment and control groups and dosing initiated (Day 0, 8-10 subjects in each group); animals in all studies followed individually throughout each experiment. Initial dosing began Day 0; animals in all groups were dosed by weight (0.01 mL per gram; 10 ml/kg). Each group was treated with vehicle (control, p.o./q.d. to the endpoint), or RAD1901 (30 or 60 mg/kg of the subject, p.o./q.d. to the endpoint) as specified from day 0. Animals in RAD1901 60 mg/kg group were taken done on Day 32 due to weakness and heavy body weight loss. The treatment period of the remained animals (vehicle and RAD1901 30 mg/kg groups) lasted up to 42 days.

### Tumor Measurements

Tumor sizes were measured twice per week in two dimensions using a caliper, and the volume was expressed in mm³ using the formula: V = 0.5 *a* x *b²* where *a* and *b* were the long and short diameters of the tumor, respectively. The tumor size was then used for calculations of tumor growth inhibition (TGI) value.

TGI was calculated for each group using the formula: TGI (%) = [1-(Ti-T0)/ (Vi-V0)] × 100; Ti is the average tumor volume of a treatment group on a given day, TO is the average tumor volume of the treatment group on the day of grouping, Vi is the average tumor volume of the vehicle control group on a given day with Ti, and V0 is the average tumor volume of the vehicle group on the day of grouping.

### Statistical Analysis

Summary statistics, including mean and the standard error of the mean (SEM), are provided for the tumor volume of each group at each time point.

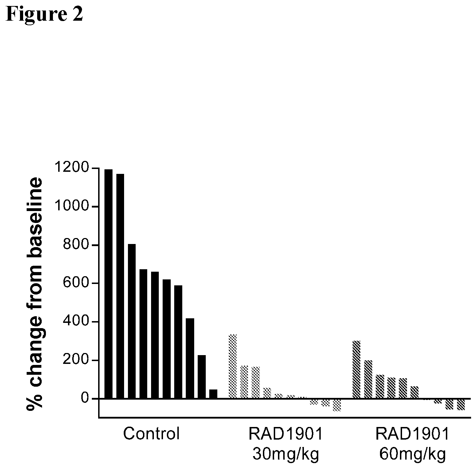

A one-way ANOVA was performed to compare tumor volume among RAD1901-treted groups compared to the vehicle-treated group on day 32 of treatment. All data were analyzed using Graphpad Prism. *p* < 0.05 was considered to be statistically significant. As can be seen in Figure 1, RAD1901 was efficacious (resulted in about 80% tumor growth inhibition, p-value >0.0001) and had approximately equal activity at both dose groups. Tumor sizes of individual animals on Day 32 show that RAD1901 resulted in tumor regressions in some animals treated in both 30 mg/kg and 60 mg/kg groups (Figure 2).

As stated above, the foregoing is merely intended to illustrate various embodiments of the present invention.

## Claims

1. A compound for use in inhibiting tumor growth or producing tumor regression in a subject having ovarian cancer or tumor selected from the group consisting of epithelial ovarian cancer, stromal ovarian cancer and ovarian sarcoma cancer, wherein said compound is RAD1901 having the structure: 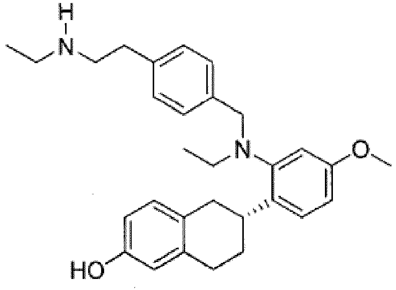 or a salt or solvate thereof.

2. The compound for use according to claim 1 wherein said ovarian cancer or tumor expresses ERα.

3. The compound for use according to claim 1 or claim 2, wherein the cancer or tumor is a metastatic cancer.

4. The compound for use according to any one of claims 1-3 wherein RAD1901 is administered at a daily oral dose of between 100 and 1,000 mg, optionally where the daily dose is 400 mg.

5. The compound for use according to any one of claims 1-4, wherein the amount of said compound that is administered is 150 mg to 2,000 mg, optionally wherein the amount of said compound that is administered is 200 mg, 400 mg, or 500 mg.

6. The compound for use according to any one of claims 1-5 wherein said use further comprises the administration of a taxane.

7. The compound for use according to any one of claims 1 to 6, wherein the ovarian cancer or tumor is resistant to a drug selected from the group consisting of anti-estrogens, aromatase inhibitors, and combinations thereof.

8. The compound for use according to claim 7, wherein the anti-estrogen is tamoxifen or fulvestrant.

9. The compound for use according to claim 7, wherein the aromatase inhibitors is aromasin.

10. The compound for use according to any one of claims 1-9, further comprising administering to the subject a therapeutically effective amount of one or more second therapeutic agents selected from the group consisting of CDK4 and/or CDK6 inhibitors, optionally wherein the CDK4 and/or CDK6 inhibitors are selected from the group consisting of ribociclib, abemaciclib and palbociclib.

11. The compound for use according to any one of claims 1 to 10, wherein one or more ovarian cancer or tumor of the subject has been surgically removed before the initiation of the administration of RAD1901.

12. The compound for use according to any one of claims 1 to 11, wherein the subject has an elevated risk of having ovarian cancer or tumor, and the administration of RAD1901 reduces the chances of the subject getting the ovarian cancer or tumor.

## Patentansprüche

1. Verbindung zur Verwendung bei einem Hemmen von Tumorwachstum oder einem Hervorrufen von Tumorregression in einem Subjekt, das einen Eierstockkrebs oder - tumor aufweist, der aus der Gruppe ausgewählt ist, die aus epithelialem Eierstockkrebs, Stroma-Eierstockkrebs und Eierstocksarkomkrebs besteht, wobei die Verbindung RAD1901 ist, die die folgende Struktur aufweist: 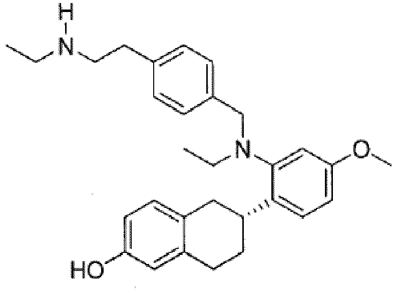 oder ein Salz oder Solvat davon.

2. Verbindung zur Verwendung nach Anspruch 1, wobei der Eierstockkrebs oder -tumor ERa exprimiert.

3. Verbindung zur Verwendung nach Anspruch 1 oder 2, wobei der Krebs oder der Tumor ein metastatischer Krebs ist.

4. Verbindung zur Verwendung nach einem der Ansprüche 1-3, wobei RAD1901 in einer oralen Tagesdosis zwischen 100 und 1.000 mg verabreicht wird, optional wenn die Tagesdosis 400 mg beträgt.

5. Verbindung zur Verwendung nach einem der Ansprüche 1-4, wobei die Menge der Verbindung, die verabreicht wird, 150 mg bis 2.000 mg beträgt, optional wobei die Menge der Verbindung, die verabreicht wird, 200 mg, 400 mg oder 500 mg beträgt.

6. Verbindung zur Verwendung nach einem der Ansprüche 1-5, wobei das Verwenden ferner die Verabreichung eines Taxans umfasst.

7. Verbindung zur Verwendung nach einem der Ansprüche 1 bis 6, wobei der Eierstockkrebs oder -tumor resistent gegen ein Arzneimittel ist, das aus der Gruppe ausgewählt ist, die aus Antiöstrogenen, Aromatasehemmern und Kombinationen davon besteht.

8. Verbindung zur Verwendung nach Anspruch 7, wobei das Antiöstrogen Tamoxifen oder Fulvestrant ist.

9. Verbindung zur Verwendung nach Anspruch 7, wobei der Aromatasehemmer Aromasin ist.

10. Verbindung zur Verwendung nach einem der Ansprüche 1-9, die ferner das Verabreichen einer therapeutisch wirksamen Menge eines oder mehrerer zweiter therapeutischer Mittel an das Subjekt Inumfasst, die aus der Gruppe ausgewählt sind, die aus CDK4- und/oder CDK6-Hemmstoffen besteht, optional wobei die CDK4- und/oder CDK6-Hemmstoffe aus der Gruppe ausgewählt sind, die aus Ribociclib, Abemaciclib und Palbociclib besteht.

11. Verbindung zur Verwendung nach einem der Ansprüche 1 bis 10, wobei ein oder mehrere Eierstockkrebs oder -tumore des Subjekts vor dem Beginn der Verabreichung von RAD1901 chirurgisch entfernt wurden.

12. Verbindung zur Verwendung nach einem der Ansprüche 1 bis 11, wobei das Subjekt ein erhöhtes Risiko aufweist, an einem Eierstockkrebs oder -tumor erkrankt zu sein, und die Verabreichung von RAD1901 die Wahrscheinlichkeit verringert, dass das Subjekt an einem Eierstockkrebs oder -tumor erkrankt.

## Revendications

1. Composé destiné à être utilisé pour inhiber la croissance tumorale ou produire une régression tumorale chez un sujet atteint d'un cancer ou d'une tumeur de l'ovaire choisie dans le groupe constitué par le cancer épithélial de l'ovaire, le cancer de l'ovaire stromal et le cancer du sarcome de l'ovaire, ledit composé étant le RAD1901 ayant la structure suivante : 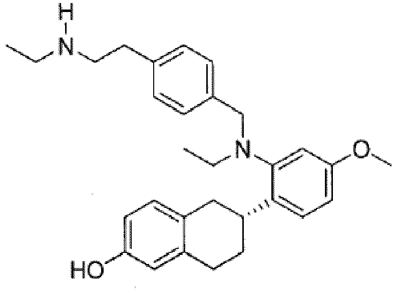 ou un sel ou solvate de celui-ci.

2. Composé pour utilisation selon la revendication 1, dans lequel ledit cancer ou la tumeur de l'ovaire exprime l'ERα.

3. Composé destiné à être utilisé selon la revendication 1 ou la revendication 2, dans lequel le cancer ou la tumeur est un cancer métastatique.

4. Composé destiné à être utilisé selon l'une quelconque des revendications 1 à 3, dans lequel RAD1901 est administré à une dose orale quotidienne comprise entre 100 et 1 000 mg, éventuellement lorsque la dose quotidienne est de 400 mg.

5. Composé destiné à être utilisé selon l'une quelconque des revendications 1 à 4, dans lequel la quantité dudit composé qui est administré est de 150 à 2 000 mg, éventuellement dans lequel la quantité dudit composé qui est administré est de 200 mg, 400 mg ou 500 mg.

6. Composé destiné à être utilisé selon l'une quelconque des revendications 1 à 5, dans lequel ladite utilisation comprend en outre l'administration d'un taxane.

7. Composé destiné à être utilisé selon l'une quelconque des revendications 1 à 6, dans lequel le cancer ou la tumeur de l'ovaire est résistant à un médicament choisi dans le groupe constitué par les anti-œstrogènes, les inhibiteurs de l'aromatase et leurs combinaisons.

8. Composé pour utilisation selon la revendication 7, dans lequel anti-œstrogène est un taximophène ou un fulvestrant.

9. Composé pour utilisation selon la revendication 7, dans lequel les inhibiteurs d'aromatase est l'aromasine.

10. Composé destiné à être utilisé selon l'une quelconque des revendications 1 à 9, comprenant en outre l'administration au sujet d'une quantité thérapeutiquement efficace d'un ou plusieurs seconds agents thérapeutiques choisis dans le groupe constitué des inhibiteurs de CDK4 et/ou de CDK6, éventuellement dans lequel les inhibiteurs de CDK4 et/ou de CDK6 sont choisis dans le groupe constitué par le ribociclib, l'abémaciclib et le palbociclib.

11. Composé destiné à être utilisé selon l'une quelconque des revendications 1 à 10, dans lequel un ou plusieurs cancers ou tumeurs de l'ovaire du sujet ont été éliminés chirurgicalement avant le début de l'administration de RAD1901.

12. Composé destiné à être utilisé selon l'une quelconque des revendications 1 à 11, dans lequel le sujet présente un risque élevé d'avoir un cancer ou une tumeur de l'ovaire, et l'administration de RAD1901 réduit les chances du sujet de contracter le cancer ou la tumeur de l'ovaire.
